(19) Europäisches Patentamt
European Patent Office
Office européen des brevets

(11) Numéro de publication : **0 302 788 B1**

# (12) FASCICULE DE BREVET EUROPEEN

(45) Date de publication du fascicule du brevet : **05.02.92 Bulletin 92/06**

(21) Numéro de dépôt : **88402024.9**

(22) Date de dépôt : **03.08.88**

(51) Int. Cl.$^5$ : **C07D 471/04,** A61K 31/445, // (C07D471/04, 221:00, 209:00)

## (54) Dérivés de [(pipéridinyl-4)méthyl]-2 tétrahydro-1,2,3,4 9H-pyrido[3,4-b]indole, leur préparation et leur application en thérapeutique.

(30) Priorité : **07.08.87 FR 8711291**

(43) Date de publication de la demande : **08.02.89 Bulletin 89/06**

(45) Mention de la délivrance du brevet : **05.02.92 Bulletin 92/06**

(84) Etats contractants désignés : **AT BE CH DE ES FR GB GR IT LI LU NL SE**

(56) Documents cités : **US-A- 4 663 456**

(73) Titulaire : **SYNTHELABO**
**58 rue de la Glacière**
**F-75013 Paris (FR)**

(72) Inventeur : **Sevrin, Mireille**
**73, rue Raymond Losserand**
**F-75014 Paris (FR)**
Inventeur : **George, Pascal**
**19, rue des Quatre Vents**
**F-78730 St Arnoult en Yveslines (FR)**
Inventeur : **Menin, Jacques**
**Cité Balzac - D 114 - 118, rue Balzac**
**F-94400 Vitry sur Seine (FR)**
Inventeur : **Morel, Claude**
**25, rue Gabriel Péri - Cresly**
**F-78470 Magny les Hameaux (FR)**
Inventeur : **Bigg, Dennis**
**122, avenue de Lavaur**
**81100 Castres (FR)**

(74) Mandataire : **Ludwig, Jacques et al**
**SYNTHELABO, Service Brevets, 22, avenue Galilée, B.P. 72**
**F-92352 Le Plessis Robinson Cédex (FR)**

EP 0 302 788 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet des dérivés de [(pipéridinyl-4)]méthyl-2 tétrahydro-1,2,3,4 9H-pyrido[3,4-b]indole, leur préparation et leur application en thérapeutique.

Les composés de l'invention répondent à la formule générale (I)

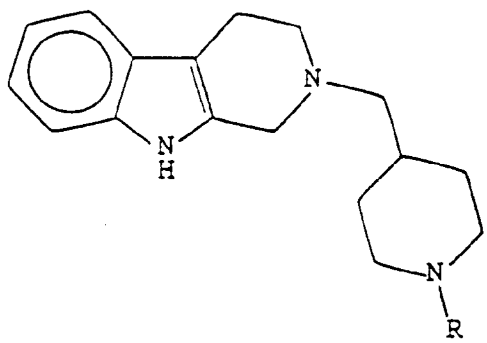

(I)

dans laquelle R représente soit un atome d'hydrogène, soit un groupe alkylcarbonyle, arylalkylcarbonyle ou arylcarbonyle de formule générale $COR_1$ où $R_1$ représente un groupe alkyle en $C_1$-$C_6$ ou un groupe benzyle ou un groupe phényle portant éventuellement de 1 à 3 substituants choisis parmi les atomes d'halogènes et les groupes trifluorométhyle, alkyles en $C_1$-$C_3$ et alcoxy en $C_1$-$C_3$, soit un groupe alcoxycarbonyle ou benzyloxycarbonyle de formule générale $COOR_2$ où $R_2$ représente un groupe alkyle en $C_1$-$C_6$ ou un groupe benzyle, soit un groupe aminocarbonyle substitué de formule générale $CONHR_3$ où $R_3$ représente un groupe alkyle en $C_1$-$C_6$ ou un groupe phényle, soit un groupe arylsulfonyle de formule générale $SO_2R_4$ où $R_4$ représente un groupe phényle.

Ils peuvent exister à l'état de bases libres ou de sels d'addition à des acides.

Conformément à l'invention on peut préparer les composés de formule générale (I) selon des procédés illustrés par les schémas qui vont suivre.

Selon une première variante, illustrée par le schéma 1, on fait d'abord réagir l'acide pipéridine-4-carboxylique de formule (II) avec le chloroformiate de benzyle, en présence d'une base et en milieu aqueux, pour obtenir l'acide (benzyloxycarbonyl)-1 pipéridine-4-carboxylique de formule (III), puis on fait réagir le tétrahydro-1,2,3,4 9H-pyrido[3,4-b]-

Schéma 1

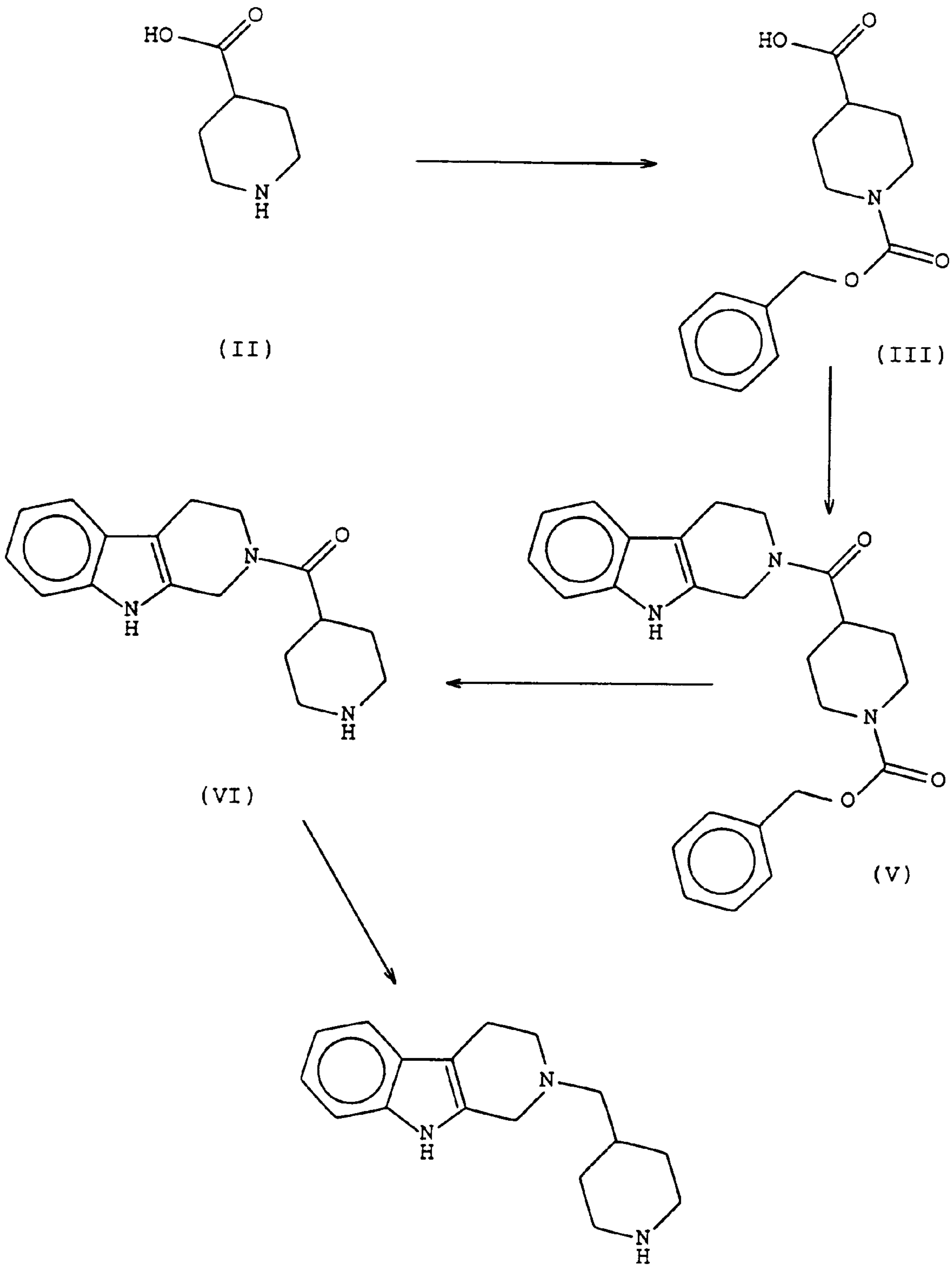

(II)

(III)

(VI)

(V)

(VII)

Schéma 2

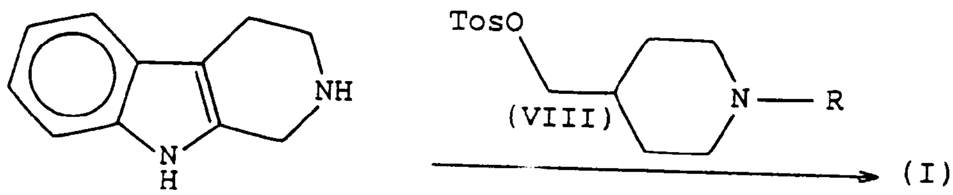

(VIII)

(I)

Schéma 3

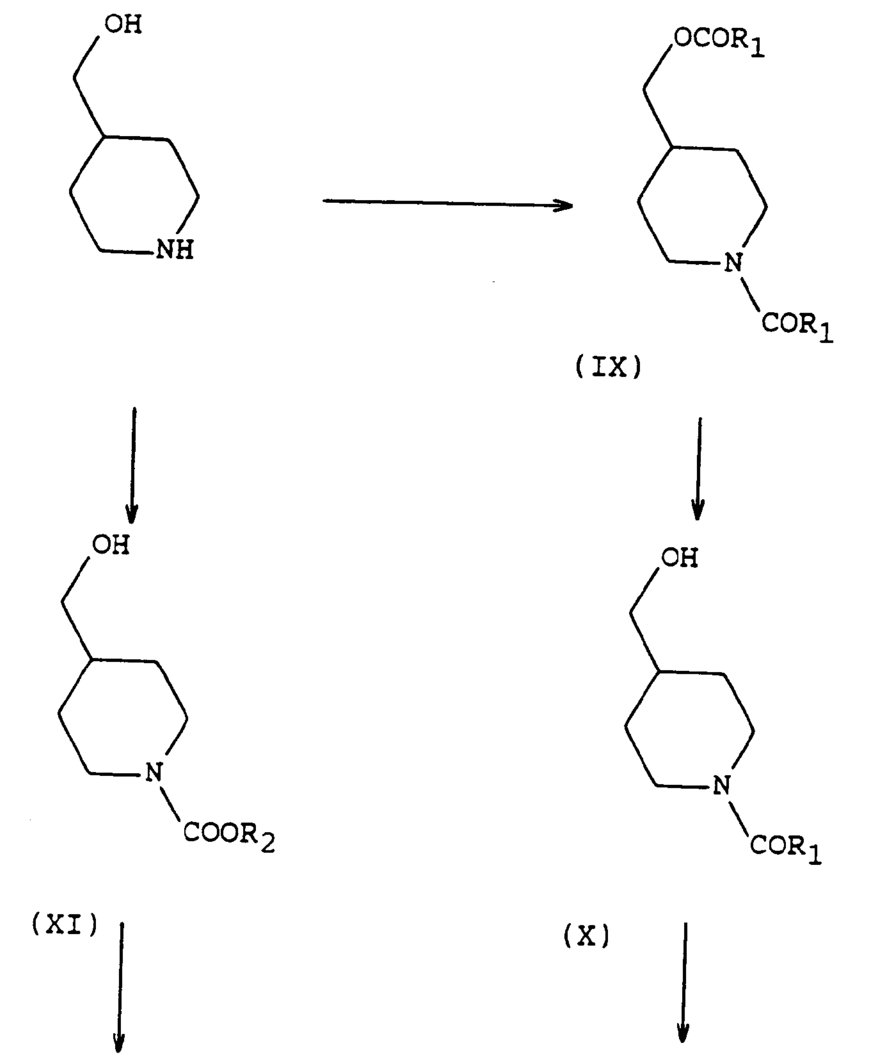

(IX)

(XI)

(X)

(VIII, R = $COOR_2$)

(VIII, R = $COR_1$)

indole avec le chlorure d'acide du composé de formule (III) (préparé in situ au moyen d'un agent connu à cet effet, par exemple le chlorure de thionyle), dans un solvant inerte, par exemple le tétrahydrofuranne, en présence d'une base, par exemple la triéthylamine, à température ambiante, pour obtenir le [(tétrahydro-1,2,3,4 9H-pyrido[3,4-b]indolyl-2)-carbonyl]-4 pipéridine-1-carboxylate de benzyle de formule (V), que l'on soumet à une réduction catalytique pour obtenir le [pipéridinyl-4-carbonyl]-2 tétrahydro-1,2,3,4 9H-pyrido [3,4-b]indole de formule (VI), puis on soumet ce dernier à une réduction au moyen d'hydrure de lithium et d'aluminium dans un solvant éthéré, par exemple l'éther diéthylique ou le tétrahydrofuranne, à une température de 20 à 67°C, pour obtenir le [(pipéridinyl-4)méthyl]-2 tétrahydro-1,2,3,4 9H-pyrido[3,4-b]indole de formule (VII).

Ce composé de formule (VII) correspond au composé de formule générale (I) où R représente un atome d'hydrogène. Il sert de composé de départ pour préparer les autres composés de formule générale (I) où R est différent d'un atome d'hydrogène. Pour préparer un composé de formule générale (I) où R représente un groupe de formule générale $COR_1$, on fait réagir le [(pipéridinyl-4)méthyl]-2 tétrahydro-1,2,3,4 9H-pyrido-[3,4-b]indole de formule (VII) avec un chlorure d'acide de formule générale $ClCOR_1$ (où $R_1$ est tel que défini ci-dessus), en présence d'une base, par exemple la triéthylamine, dans un solvant halogéné, par exemple le chloroforme, à température ambiante.

Pour préparer un composé de formule générale (I) où R représente un groupe de formule générale $COOR_2$, on fait réagir le [(pipéridinyl-4)méthyl]-2 tétrahydro-1,2,3,4 9H-pyrido-[3,4-b]indole de formule (VII) avec un haloformiate, par exemple un chloroformiate de formule générale $ClCOOR_2$ (où $R_2$ est tel que défini ci-dessus), en présence d'une base, par exemple la triéthylamine, dans un solvant halogéné, par exemple le chloroforme, à température ambiante. Pour préparer un composé de formule générale (I) où R représente un groupe de formule générale $CONHR_3$, on fait réagir le [(pipéridinyl-4)méthyl]-2 tétrahydro-1,2,3,4 9H-pyrido-[3,4-b]indole de formule (VII) avec un isocyanate de formule générale $R_3NCO$ (où $R_3$ est tel que défini ci-dessus), dans un solvant halogéné, par exemple le chloroforme. Pour préparer un composé de formule générale (I) où R représente un groupe de formule générale $SO_2R_4$, on fait réagir le [(pipéridinyl-4)méthyl]-2 tétrahydro-1,2,3,4 9H-pyrido-[3,4-b]indole de formule (VII) avec le chlorure de phénylsulfonyle de formule générale $ClSO_2R_4$ (où $R_4$ est tel que défini ci-dessus), en présence d'une base, par exemple la triéthylamine, dans un solvant halogéné, par exemple le chloroforme ou le dichlorométhane, à température ambiante.

Lorsque R représente un groupe de formule générale $COR_1$ ou $COOR_2$, on peut utiliser une seconde variante de procédé, illustrée par le schéma 2 qui précède. Cette variante consiste à faire réagir le tétrahydro-1,2,3,4 9H-pyrido[3,4-b]indole avec un tosylate de formule générale (VIII) (dans laquelle Tos représente un groupe tosyle et R représente un groupe de formule générale $COR_1$ ou $COOR_2$) soit en l'absence soit en présence d'un solvant inerte tel que le diméthylformamide ou le xylène, à une température de 20 à 150°C, et éventuellement en présence d'une base organique, telle qu'une amine tertiaire, ou minérale telle qu'un carbonate ou hydrogénocarbonate alcalin.

Le tosylate de formule générale (VIII) peut être préparé selon la méthode suivante, illustrée par le schéma 3. Lorsque R représente un groupe de formule générale $COR_1$, on fait réagir le pipéridine-4-méthanol avec un chlorure d'acide de formule générale $ClCOR_1$, dans un solvant inerte tel qu'un solvant chloré, à une température de 20 à 80°C. On obtient ainsi un ester-amide de formule générale (IX), qu'on saponifie, par exemple au moyen d'hydroxyde de sodium ou de potassium, dans un solvant alcoolique aliphatique inférieur, de préférence l'éthanol, pour obtenir l'alcool de formule générale (X), dont on prépare finalement le tosylate au moyen de chlorure de tosyle, dans un milieu basique tel que la pyridine.

Lorsque R représente un groupe de formule générale $COOR_2$ on fait réagir le pipéridine-4-méthanol avec un chloroformiate de formule générale $ClCOOR_2$ dans un solvant tel qu'un solvant chloré, à température ambiante. On obtient ainsi un carbamate de formule générale (XI) dont on prépare le tosylate comme indiqué précédemment.

Le pipéridine-4-méthanol peut être obtenu par exemple par réduction de pipéridine-4-carboxylate d'éthyle au moyen d'hydrure de lithium et aluminium, ou encore par une telle réduction de benzyl-1 pipéridine-4-carboxylate d'éthyle suivie d'une hydrogénolyse catalytique sous pression.

Les exemples suivants illustrent en détail la préparation de quelques composés selon l'invention. Les microanalyses élémentaires et les spectres IR et RMN confirment les structures des produits obtenus.
Les numéros indiqués entre parenthèses dans les titres des exemples correspondent à ceux du tableau donné plus loin.

Exemple 1 (Composé N° 1)

[(Pipéridinyl-4)méthyl]-2 tétrahydro-1,2,3,4 9H-pyrido[3,4-b] indole, dichlorhydrate.

1.1. Acide (benzyloxycarbonyl)-1 pipéridine-4-carboxylique.

Sous atmosphère d'argon on introduit dans un ballon 51,6 g (0,4 mole) d'acide pipéridine-4-carboxylique,

600 ml d'eau et 35,2 g (0,88 mole) d'hydroxyde de sodium en paillettes. On refroidit la solution à 5°C, on ajoute rapidement 68,6 ml (0,48 mole) de chloroformiate de benzyle, on agite le mélange pendant 1 h à 0°C puis pendant 2 h à 20°C, et on le traite avec 250 ml d'eau. On lave la phase aqueuse deux fois avec 200 ml de toluène, on ajoute de l'acide acétique et on l'extrait avec du dichlorométhane. On sépare la phase organique, on la lave à l'eau, on la sèche sur sulfate de sodium et on évapore le solvant sous pression réduite. L'huile résiduelle cristallise dans l'hexane. On obtient 100 g de produit sec.
Point de fusion : 78-80°C.

1.2. [(Tétrahydro-1,2,3,4 9H-pyrido[3,4-b]indolyl-2)carbonyl]-4 pipéridine-1-carboxylate de benzyle.

A une solution de 25 g (95 mmoles) d'acide (benzyloxycarbonyl)-1 pipéridine-4-carboxylique dans 500 ml de toluène on ajoute, sous atmosphère d'argon, 25 ml (343 mmoles) de chlorure de thionyle. On chauffe le mélange au reflux pendant 5 h puis on évapore le solvant sous pression réduite. On reprend le résidu avec 500 ml de toluène et on l'évapore de nouveau. On obtient 25,5 g de produit huileux qu'on dissout dans 250 ml de tétrahydrofuranne, on ajoute cette solution, sous atmosphère d'argon, à une solution de 16,6 g (90 mmoles) de tétrahydro-1,2,3,4 9H-pyrido[3,4-b]indole dans 800 ml de tétrahydrofuranne et 12,7 ml (90 mmoles) de triéthylamine, et on agite le mélange à 20°C pendant 48 h. On sépare un précipité par filtration, on le lave au tétrahydrofuranne, on évapore le filtrat sous pression réduite, on dissout le résidu dans 1200 ml de dichlorométhane, on lave la solution avec une solution aqueuse 1 N d'hydroxyde de sodium, puis avec de l'eau, on la sèche sur sulfate de sodium et on évapore le solvant sous pression réduite. On obtient 32 g d'un solide blanc.
Point de fusion : 160-162°C.

1.3. [(Pipéridinyl-4)carbonyl]-2 tétrahydro-1,2,3,4 9H-pyrido [3,4-b]indole.

On introduit 32 g (76,65 mmoles) de [(tétrahydro-1,2,3,4 9H-pyrido[3,4-b]indolyl-2)carbonyl]-4 pipéridine-1-carboxylate de benzyle, 300 ml de méthanol, 300 ml de dichlorométhane et 9 g de charbon palladié à 10% dans un appareil de Parr et on effectue une hydrogénolyse sous environ 0,41 MPa pendant 18 h.

On filtre le mélange réactionnel, on évapore le filtrat et on reprend le résidu avec 1000 ml d'eau. On ajoute de l'hydroxyde de sodium jusqu'à pH basique et on extrait la phase aqueuse avec du dichlorométhane. On sépare la phase organique, on la lave à l'eau, on la sèche sur sulfate de sodium, on évapore le solvant sous pression réduite et on recristallise le résidu dans de l'acétate d'éthyle. On obtient 17,2 g d'un solide blanc.
Point de fusion : 190-192°C.

1.4. [(Pipéridinyl-4)méthyl]-2 tétrahydro-1,2,3,4 9H-pyrido [3,4-b]indole, dichlorhydrate.

A une suspension de 6 g (158 mmoles) d'hydrure de lithium et d'aluminium dans 150 ml de tétrahydrofuranne on ajoute, sous atmosphère d'argon, 6 g (21,17 mmoles) de [(pipéridinyl-4)carbonyl]-2 tétrahydro-1,2,3,4 9H-pyrido[3,4-b]indole en solution dans 800 ml de tétrahydrofuranne, et on chauffe le mélange au reflux pendant 12 h. On l'hydrolyse à 0°C avec 6,5 ml d'eau, 5 ml d'une solution aqueuse à 20% d'hydroxyde de sodium, et 22 ml d'eau.

On filtre le mélange, on évapore le solvant sous pression réduite, on met le résidu en suspension dans 500 ml d'eau, on le filtre, on le sèche et on le recristallise dans le minimum d'éthanol. On obtient ainsi 2,37 g de base libre. Point de fusion : 189-190°C.
On en prépare le dichlorhydrate dans l'alcool isopropylique chlorhydrique O,1N.
Point de fusion : 290-292°C.

Exemple 2 (Composé N° 2)

[(Benzoyl-1 pipéridinyl-4-méthyl]-2 tétrahydro-1,2,3,4 9H-pyrido[3,4-b]indole.

Sous atmosphère d'argon on introduit dans un ballon de Keller 2,7 g (10 mmoles) de [(pipéridinyl-4)méthyl]-2 tétrahydro-1,2,3,4 9H-pyrido[3,4-b]indole, 30 ml de chloroforme et 1,6 ml (11 mmoles) de triéthylamine. On ajoute 1,546 g (11 mmoles) de chlorure de benzoyle en solution dans 100 ml de chloroforme et on agite le mélange à 20°C pendant 48 h. On évapore le solvant sous pression réduite, on reprend le résidu avec de l'eau, on le filtre, on le dissout dans de l'acétate d'éthyle et on le traite avec du charbon activé. Après filtration on évapore le solvant sous pression réduite, on fait cristalliser le résidu dans l'éther isopropylique et on le recristallise dans l'acétonitrile. On obtient 1,8 g de solide blanc. Point de fusion : 161,5-163,5°C.

Exemple 3 (Composé N° 4)

[(Tétrahydro-1,2,3,4 9H-pyrido[3,4-b]indolyl-2)méthyl]-4 pipéridine-1-carboxylate d'éthyle.

Sous atmosphère d'argon on introduit dans un ballon de Keller 7,06 g (26,2 mmoles de [(pipéridinyl-4)méthyl]-2 tétrahydro-1,2,3,4 9H-pyrido[3,4-b]indole, 70 ml de chloroforme, 4 ml (26,2 mmoles) de triéthylamine, et on ajoute 2,8 ml (26,2 mmoles) de chloroformiate d'éthyle.

On agite le mélange pendant 14 h à 20°C, on évapore le solvant sous pression réduite, on reprend le résidu avec de l'eau et on extrait la phase aqueuse avec de l'acétate d'éthyle. On sépare la phase organique, on la sèche sur sulfate de sodium, on évapore le solvant sous pression réduite et on recristallise le résidu dans l'acétonitrile. On obtient 2 g d'un solide blanc.
Point de fusion : 117,5-120°C.

Exemple 4 (Composé N° 6)

N-phényl [(tétrahydro-1,2,3,4 9H-pyrido[3,4-b]indolyl-2) méthyl]-4 pipéridine-1-carboxamide.

Sous atmosphère d'argon on introduit dans un ballon 7,5 g (27,7 mmoles) de [(pipéridinyl-4)méthyl]-2 tétrahydro-1,2,3,4 9H-pyrido[3,4-b]indole et 75 ml de chloroforme, on ajoute 7,5 ml (67,6 mmoles) d'isocyanate de phényle et on agite le mélange pendant 14 h à 20°C. On filtre le précipité qui s'est formé, on le rince à l'éther isopropylique et on le recristallise dans le méthanol. On obtient 7,5 g de produit.
Point de fusion : 207-209°C.

Exemple 5 (Composé N° 7)

[(Phénylsulfonyl-1 pipéridinyl-4)méthyl]-2 tétrahydro-1,2,3,4 9H-pyrido[3,4-b]indole.

A une suspension de 2,7 g (10 mmoles) de [(pipéridinyl-4)méthyl]-2 tétrahydro-1,2,3,4 9H-pyrido[3,4-b]indole et 1,6 ml (10 mmoles) de triéthylamine dans 30 ml de chloroforme on ajoute 1,4 ml (11 mmoles) de chlorure de phénylsulfonyle. On obtient une solution qu'on agite à 20°C pendant 24 h.

On évapore le solvant sous pression réduite, on reprend le résidu avec de l'eau, on extrait la phase aqueuse avec du dichlorométhane, on sèche la phase organique sur sulfate de sodium, on évapore le solvant sous pression réduite, et on recristallise le résidu dans de l'acétate d'éthyle. On obtient 0,86 g d'un solide blanc.
Point de fusion : 142-145°C.

Exemple 6 (Composé N° 9)

[[(Méthyl-3 benzoyl)-1 pipéridinyl-4]méthyl]-2 tétrahydro-1,2,3,4 9H-pyrido[3,4-b]indole.

6.1 Pipéridine-4-méthanol.

Dans un ballon tricol de 4 l muni d'un système d'agitation mécanique et d'un réfrigérant on introduit 28,5 g (0,75 mole) d'hydrure de lithium et aluminium et 1,2 l de tétrahydrofuranne. On ajoute à la suspension obtenue 117,9 g (0,75 mole) de pipéridine-4-carboxylate d'éthyle en solution dans 1,2 l de tétrahydrofuranne, et on agite le mélange pendant 6 h à 20°C. On le refroidit à 0°C, puis on l'hydrolyse en ajoutant successivement 22 ml d'eau, 22 ml d'hydroxyde de sodium 1 N et 46 ml d'eau. On agite le mélange 30 mn à 20°C, on le filtre, on lave le précipité au tétrahydrofuranne puis à l'éther. On évapore les solvants sous pression réduite, et on obtient 84,4 g d'une huile qu'on utilise telle quelle dans l'étape suivante.

6.2. Méthyl-3 benzoate de [(méthyl-3 benzoyl)-1 pipéridinyl-4]méthyle.

Dans un ballon tricol de 3 l on introduit, sous atmosphère d'argon, 42,25 g (0,367 mole) de pipéridine-4-méthanol, 430 ml de dichloro-1,2 éthane, et on ajoute 82 g (0,81 mole) de triéthylamine puis 125,2 g (0,81 mole) de chlorure de méthyl-3 benzoyle. On chauffe le mélange au reflux pendant 4 h 30, on ajoute encore 8,2 g (0,08 mole) de triéthylamine et 12,5 g (0,08 mole) de chlorure de méthyl-3 benzoyle, et on chauffe le mélange pendant encore 3 h.

On le filtre, on lave les sels au dichloro-1,2 éthane, on évapore le filtrat sous pression réduite, on dissout le résidu dans de l'acétate d'éthyle, on lave la solution avec une solution aqueuse saturée de chlorure de

sodium, on évapore le solvant sous pression réduite, et on recristallise le résidu dans un mélange 1/1 d'alcool isopropylique/acétate d'éthyle. On obtient 80 g d'un solide blanc.
Point de fusion : 80-83°C.

6.3. (Méthyl-3 benzoyl)-1 pipéridine-4-méthanol.

A une solution de 80 g (0,23 mole) de méthyl-3 benzoate de [(méthyl-3 benzoyl)-1 pipéridinyl-4]méthyle dans 400 ml d'éthanol, on ajoute une solution de 12,76 g (0,23 mole) d'hydroxyde de potassium dans 75 ml d'éthanol et 75 ml d'eau. On agite le mélange à 20°C pendant 3 h, on évapore le solvant sous pression réduite et on extrait la phase aqueuse à l'acétate d'éthyle. On lave la phase organique avec de l'eau puis avec une solution aqueuse saturée de chlorure de sodium, et on la sèche sur sulfate de magnésium. On évapore le solvant sous pression réduite et on obtient 53 g d'alcool qu'on utilise tel quel dans l'étape suivante.

6.4. Méthyl-4 benzènesulfonate de [(méthyl-3 benzoyl)-1 pipéridinyl-4]méthyle.

A une solution de 52 g (0,22 mole) de (méthyl-3 benzoyl)-1 pipéridine-4-méthanol dans 100 ml de pyridine on ajoute 53,3 g (0,28 mole) de chlorure de méthyl-4 benzènesulfonyle dans 60 ml de pyridine. On agite le mélange à 20°C pendant 4 h, puis on le verse dans de la glace. On extrait la phase aqueuse au dichlorométhane, on lave la phase organique avec une solution aqueuse 10 N d'acide chlorhydrique et on la sèche sur sulfate de magnésium. On évapore les solvants sous pression réduite et on obtient 70 g de solide blanc.
Point de fusion : 68-70°C.

6.5. [[(Méthyl-3 benzoyl)-1 pipéridinyl-4]méthyl]-2 tétrahydro-1,2,3,4 9H-pyrido[3,4-b]indole.

Sous atmosphère d'argon on introduit dans un ballon de 100 ml 1,7 g (10 mmoles) de tétrahydro-1,2,3,4 9H-pyrido[3,4-b]indole, 3,9 g (10 mmoles) de méthyl-4 benzènesulfonate de [(méthyl-3 benzoyl)-1 pipéridinyl-4]méthyle, 2,9 g (20 mmoles) de carbonate de potassium et 25 ml de diméthylformamide. On agite le mélange à 100°C pendant 5 h, on l'hydrolyse, et on extrait la phase aqueuse avec de l'acétate d'éthyle. On lave la phase organique à l'eau, puis avec une solution aqueuse saturée de chlorure de sodium, on la sèche sur sulfate de sodium et on évapore les solvants sous pression réduite. On purifie le résidu par chromatographie sur colonne de silice en éluant avec un mélange 9/1 de dichlorométhane/méthanol et, après recristallisation dans l'acétate d'éthyle, on obtient 1,2 g de base pure.
Point de fusion : 185-187°C.

Le tableau suivant illustre les structures et points de fusions de quelques composés selon l'invention.

Tableau

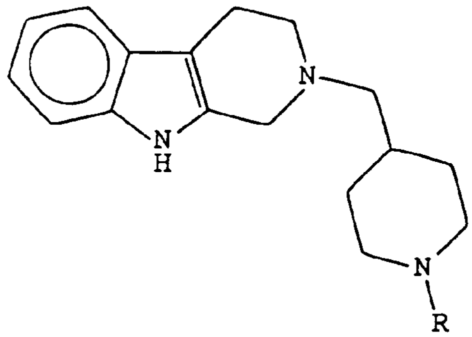

(I)

| N° | R | Sel ou base | F(°C) |
|---|---|---|---|
| 1 | H | dichlorhydrate | 290-292 |
| 2 | $COC_6H_5$ | base | 161,5-163,5 |
| 3 | $COCH_2C_6H_5$ | benzènesulfonate | 197-199 |
| 4 | $COOC_2H_5$ | base | 117,5-120 |
| 5 | $CONHnC_3H_7$ | base | 207-209,5 |
| 6 | $CONHC_6H_5$ | base | 207-209 |
| 7 | $SO_2C_6H_5$ | base | 142-145 |
| 8 | $COC_6H_4$-3-Cl | base | 199-201 |
| 9 | $COC_6H_4$-3-$CH_3$ | base | 185-187 |
| 10 | $COC_6H_4$-3-$OC_2H_5$ | base | 206-208 |
| 11 | $COC_6H_4$-3-F | chlorhydrate | 228-230 |
| 12 | $COC_6H_4$-2-$CH_3$ | chlorhydrate | 231-233 |
| 13 | $COC_6H_4$-4-$CH_3$ | base | 204-206 |
| 14 | $COC_6H_3$-3,5-$(CF_3)_2$ | chlorhydrate | 266-268 |
| 15 | $COC_6H_3$-3,5-$(CH_3O)_2$ | chlorhydrate | 165-170 |
| 16 | $CONHnC_4H_9$ | base | 215-218 |
| 17 | $COCH_3$ | base | 137-139 |
| 18 | $COOiC_3H_7$ | benzènesulfonate | 215-217 |
| 19 | $COnC_3H_7$ | base | 164-166 |
| 20 | $COOCH_3$ | benzènesulfonate | 210-212 |
| 21 | $COOCH_2C_6H_5$ | benzènesulfonate | 209-211 |

Les composés de l'invention ont été soumis à une série d'essais pharmacologiques qui ont mis en évidence leur intérêt comme substances à activités thérapeutiques.

Ainsi ils ont fait l'objet d'une étude quant à leur affinité pour les récepteurs sérotoninergiques du type 5-

$HT_{1A}$. Les composés déplacent dans l'hippocampe du rat un ligand spécifique marqué, la $[^{3}H]$-hydroxy-8 dipropylamino-2 tétraline (désignée ci-après par "$[^{3}H]$-8-OH-DPAT") décrite par Gozlan et coll., Nature, (1983), 305, 140-142.

Les animaux utilisés sont des rats mâles Sprague-Dawley de 160 à 200 g. Après décapitation on en prélève le cerveau et on excise l'hippocampe. On broie le tissu dans un appareil Ultra-Turrax Polytron pendant 30 s à la moitié de la vitesse maximale dans 10 volumes de tampon Tris 50 mM d'un pH ajusté à 7,4 avec de l'acide chlorhydrique (soit 100 mg de tissu frais par ml). On lave les tissus homogénéisés trois fois à 4°C, en les centrifugeant à chaque fois à 48000 $\times$ g et en remettant le culot en suspension pendant 10 mn dans du tampon frais refroidi. Finalement on met le dernier culot en suspension dans le tampon pour arriver à une concentration de 100 mg de tissu de départ par ml de tampon à 50 MM.

On laisse ensuite incuber à 37°C pendant 10 mn. La liaison avec la $[^{3}H]$-8-OH-DPAT est déterminée par incubation de 10 $\mu$l de suspension de membranes dans un volume final de 1 ml de tampon contenant 10 $\mu$M de pargylline.

Après l'incubation on récupère les membranes par filtration sur filtres Whatman GF/B qu'on lave trois fois avec des quantités aliquotes de 5 ml de tampon glacé. On extrait les filtres dans le liquide de scintillation et on en mesure la radioactivité par scintigraphie liquide. La liaison spécifique de la $[^{3}H]$-8-OH-DPAT est définie comme la quantité radioactive retenue sur les filtres et pouvant être inhibée par co-incubation dans la hydroxy-5 tryptamine à 10 $\mu$M. A une concentration de 1 nM de $[^{3}H]$-8-OH-DPAT la liaison spécifique représente de 70 à 80% de la radioactivité totale recupérée sur le filtre.

Pour chaque concentration de composés étudié on détermine le pourcentage d'inhibition de la liaison avec la $[^{3}H]$-8-OH- DPAT, puis la concentration $CI_{50}$, concentration qui inhibe 50% de la liaison.

Pour les composés de l'invention les $CI_{50}$ se situent entre 0,001 et 0,2 $\mu$M.

L'activité centrale des composés de l'invention a été évaluée par leurs effets sur les "pointes PGO" (ponto-géniculo-occipitales) induites par la réserpine (test PGO-R) chez le chat, selon la méthode décrite par H. Depoortere, Sleep 1976, 3rd Europ. Congr. Sleep Res., Montpellier 1976, 358-361 (Karger, Basel 1977).

On administre des doses cumulatives de composés à étudier (de 0,01 à 3 mg/kg par voie intraveineuse) à des intervalles de temps de 30 mn, 4 h après injection intrapéritonéale d'une dose de 0,75 mg/kg de réserpine, à des chats curarisés, sous ventilation artificielle. On recueille les activités électroencéphalographique et phasique (pointes PGO-R) à l'aide d'électrodes corticales et profondes (genouillé latéral). Pour chaque dose de composé étudié on détermine le pourcentage de diminution du nombre de pointes PGO, puis la $DA_{50}$, dose active qui diminue de 50% ce nombre de pointes. Pour les composés de l'invention les $DE_{50}$ se situent entre 0,01 et 1 mg/kg par la voie intraveineuse.

Les résultats des essais montrent que les composés de formule générale (I) possèdent, in vitro, une grande affinité et une sélectivité pour les récepteurs sérotoninergiques de type 5-$HT_{1A}$. In vivo ils montrent une activité agoniste, agoniste partielle, ou antagoniste au niveau de ces récepteurs.

Les composés de l'invention peuvent donc être utilisés pour le traitement des maladies et affections impliquant les récepteurs sérotoninergiques de type 5-$HT_{1A}$ de façon directe ou indirecte, notamment pour le traitement des états dépressifs, des états d'anxiété, des troubles du sommeil, pour la régulation de la prise de nourriture, et pour le traitement vasculaire, cardiovasculaire ou cérébrovasculaire tels que l'hypertension ou la migraine.

A cet effet ils peuvent être présentés sous toutes formes appropriées à leur administration par voie orale ou parentérale, associés à tous excipients convenables, et dosés pour permettre une posologie journalière de 1 à 1000 mg.

## Revendications

### Revendications pour les Etats contractants suivants : AT, BE, CH, DE, FR, GB, IT, LI, LU, NL, SE

1. Composés de formule générale (I)

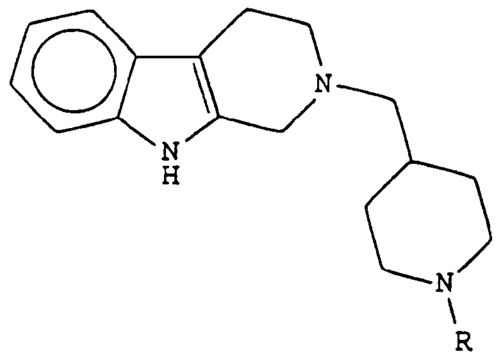

(I)

dans laquelle R représente soit un atome d'hydrogène, soit un groupe alkylcarbonyle, arylalkylcarbonyle ou arylcarbonyle de formule générale $COR_1$ où $R_1$ représente un groupe alkyle en $C_1$-$C_6$ ou un groupe benzyle ou un groupe phényle portant éventuellement de 1 à 3 substituants choisis parmi les atomes d'halogènes et les groupes trifluorométhyle, alkyles en $C_1$-$C_3$ et alcoxy en $C_1$-$C_3$, soit un groupe alcoxycarbonyle ou benzyloxycarbonyle de formule générale $COOR_2$ où $R_2$ représente un groupe alkyle en $C_1$-$C_6$ ou un groupe benzyle, soit un groupe aminocarbonyle substitué de formule générale $CONHR_3$ où $R_3$ représente un groupe alkyle en $C_1$-$C_6$ ou un groupe phényle, soit un groupe arylsulfonyle de formule générale $SO_2R_4$ où $R_4$ représente un groupe phényle,
ainsi que leurs sels d'addition à des acides acceptables en pharmacologie.

2. Procédé de préparation des composés selon la revendication 1, caractérisé en ce qu'on fait d'abord réagir l'acide pipéridine-4-carboxylique avec le chloroformiate de benzyle, en présence d'une base et en milieu aqueux, pour obtenir l'acide (benzyloxycarbonyl)-1 pipéridine-4-carboxylique, puis on fait réagir le tétrahydro-1,2,3,4 9H-pyrido[3,4-b]indole avec le chlorure d'acide (benzyloxycarbonyl)-1 pipéridine-4-carboxylique, (préparé in situ au moyen de chlorure de thionyle), dans un solvant inerte, en présence d'une base, à température ambiante, pour obtenir le [(tétrahydro-1,2,3,4 9H-pyrido[3,4-b]indolyl-2)carbonyl]-4 pipéridine-1-carboxylate de benzyle, que l'on soumet à une réduction catalytique pour obtenir le [pipéridinyl-4-carbonyl]-2 tétrahydro-1,2,3,4 9H-pyrido[3,4-b]indole, puis on soumet ce dernier à une réduction au moyen d'hydrure de lithium et d'aluminium dans un solvant éthéré, à une température de 20 à 67°C, pour obtenir le [(pipéridinyl-4)méthyl]-2 tétrahydro-1,2,3,4 9H-pyrido[3,4-b]indole, puis,

— pour préparer un composé de formule générale (I) où R représente un groupe de formule générale $COR_1$, on fait réagir le [(pipéridinyl-4)méthyl]-2 tétrahydro-1,2,3,4 9H-pyrido[3,4-b]indole avec un chlorure d'acide de formule générale $ClCOR_1$ (où $R_1$ est tel que défini dans la revendication 1), en présence d'une base, dans un solvant halogéné, à température ambiante, ou bien

— pour préparer un composé de formule générale (I) où R représente un groupe de formule générale $COOR_2$, on fait réagir le [(pipéridinyl-4)méthyl]-2 tétrahydro-1,2,3,4 9H-pyrido-[3,4-b]indole avec un chloroformiate de formule générale $ClCOOR_2$ (où $R_2$ est tel que défini dans la revendication 1), en présence d'une base, dans un solvant halogéné, à température ambiante, ou bien

— pour préparer un composé de formule générale (I) où R représente un groupe de formule générale $CONHR_3$, on fait réagir le [(pipéridinyl-4)méthyl]-2 tétrahydro-1,2,3,4 9H-pyrido[3,4-b]indole avec un isocyanate de formule générale $R_3NCO$ (où $R_3$ est tel que défini dans la revendication 1), dans un solvant halogéné, ou bien

— pour préparer un composé de formule générale (I) où R représente un groupe de formule générale $SO_2R_4$, on fait réagir le [(pipéridinyl-4)méthyl]-2 tétrahydro-1,2,3,4 9H-pyrido-[3,4-b]indole avec le chlorure de phénylsulfonyle de formule générale $ClSO_2R_4$ (où $R_4$ est tel que défini dans la revendication 1), en présence d'une base, dans un solvant halogéné, à température ambiante.

3. Procédé de préparation des composés selon la revendication 1, caractérisé en ce qu'on fait réagir le tétrahydro-1,2,3,4 9H-pyrido[3,4-b]indole avec un tosylate de formule générale (VIII)

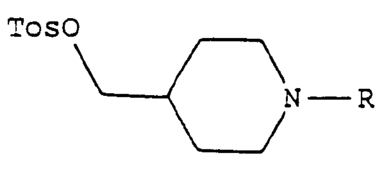

(VIII)

(dans laquelle Tos représente un groupe tosyle et R représente un groupe de formule générale $COR_1$ ou $COOR_2$) soit en l'absence soit en présence d'un solvant inerte tel que le diméthylformamide ou le xylène, à une température de 20 à 150°C, et éventuellement en présence d'une base organique, telle qu'une amine tertiaire, ou minérale telle qu'un carbonate ou hydrogénocarbonate alcalin.

4. Composition pharmaceutique caractérisée en ce qu'elle contient un composé selon la revendication 1, associé à un excipient approprié.

**Revendications pour les Etats contractants suivants : ES, GR**

1. Procédé de préparation de composés de formule générale (I)

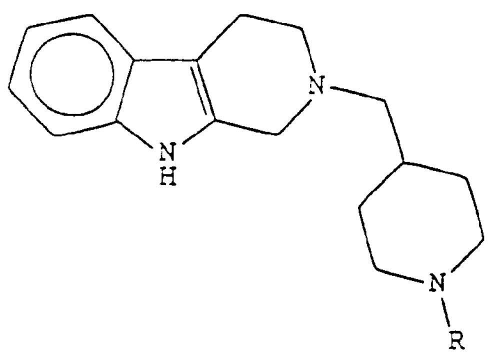

(I)

dans laquelle R représente soit un atome d'hydrogène, soit un groupe alkylcarbonyle, arylalkylcarbonyle ou arylcarbonyle de formule générale $COR_1$ où $R_1$ représente un groupe alkyle en $C_1$-$C_6$ ou un groupe benzyle ou un groupe phényle portant éventuellement de 1 à 3 substituants choisis parmi les atomes d'halogènes et les groupes trifluorométhyle, alkyles en $C_1$-$C_3$ et alcoxy en $C_1$-$C_3$, soit un groupe alcoxycarbonyle ou benzyloxycarbonyle de formule générale $COOR_2$ où $R_2$ représente un groupe alkyle en $C_1$-$C_6$ ou un groupe benzyle, soit un groupe aminocarbonyle substitué de formule générale $CONHR_3$ où $R_3$ représente un groupe alkyle en $C_1$-$C_6$ ou un groupe phényle, soit un groupe arylsulfonyle de formule générale $SO_2R_4$ où $R_4$ représente un groupe phényle,

procédé caractérisé en ce qu'on fait d'abord réagir l'acide pipéridine-4-carboxylique avec le chloroformiate de benzyle, en présence d'une base et en milieu aqueux, pour obtenir l'acide (benzyloxycarbonyle)-1 pipéridine-4-carboxylique, puis on fait réagir le tétrahydro-1,2,3,4 9H-pyrido[3,4-b]indole avec le chlorure d'acide (benzyloxycarbonyl)-1 pipéridine-4-carboxylique, (préparé in situ au moyen de chlorure de thionyle), dans un solvant inerte, en présence d'une base, à température ambiante, pour obtenir le [(tétrahydro-1,2,3,4 9H-pyrido[3,4-b]indolyl-2)carbonyl]-4 pipéridine-1-carboxylate de benzyle, que l'on soumet à une réduction catalytique pour obtenir le [pipéridinyl-4-carbonyl]-2 tétrahydro-1,2,3,4 9H-pyrido[3,4-b]indole, puis on soumet ce dernier à une réduction au moyen d'hydrure de lithium et d'aluminium dans un solvant éthéré, à une température de 20 à 67°C, pour obtenir le [(pipéridinyl-4)méthyl]-2 tétrahydro-1,2,3,4 9H-pyrido[3,4-b]indole, puis,

— pour préparer un composé de formule générale (I) où R représente un groupe de formule générale $COR_1$, on fait réagir le [(pipéridinyl-4)méthyl]-2 tétrahydro-1,2,3,4 9H-pyrido[3,4-b]indole avec un chlorure d'acide de formule générale $ClCOR_1$ (où $R_1$ est tel que défini ci-dessus), en présence d'une base, dans un solvant halogéné, à température ambiante, ou bien

— pour préparer un composé de formule générale (I) où R représente un groupe de formule générale $COOR_2$, on fait réagir le [(pipéridinyl-4)méthyl]-2 tétrahydro-1,2,3,4 9H-pyrido[3,4-b]indole avec un chloroformiate de formule générale $ClCOOR_2$ (où $R_2$ est tel que défini ci-dessus), en présence d'une base, dans un solvant halogéné, à température ambiante, ou bien

— pour préparer un composé de formule générale (I) où R représente un groupe de formule générale $CONHR_3$, on fait réagir le [(pipéridinyl-4)méthyl]-2 tétrahydro-1,2,3,4 9H-pyrido[3,4-b]indole avec un isocyanate de formule générale $R_3NCO$ (où $R_3$ est tel que défini ci-dessus), dans un solvant halogéné, ou bien

— pour préparer un composé de formule générale (I) où R représente un groupe de formule générale $SO_2R_4$, on fait réagir le [(pipéridinyl-4)méthyl]-2 tétrahydro-1,2,3,4 9H-pyrido[3,4-b]indole avec le chlorure de phénylsulfonyle de formule générale $ClSO_2R_4$ (où $R_4$ est tel que défini ci-dessus), en présence d'une base, dans un solvant halogéné, à température ambiante.

2. Procédé de préparation de composés de formule générale (I)

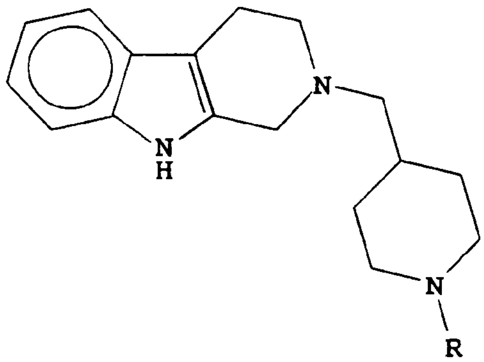

(I)

dans laquelle R représente soit un atome d'hydrogène, soit un groupe alkylcarbonyle, arylalkylcarbonyle ou arylcarbonyle de formule générale $COR_1$ où $R_1$ représente un groupe alkyle en $C_1$-$C_6$ ou un groupe benzyle ou un groupe phényle portant éventuellement de 1 à 3 substituants choisis parmi les atomes d'halogènes et les groupes trifluorométhyle, alkyles en $C_1$-$C_3$ et alcoxy en $C_1$-$C_3$, soit un groupe alcoxycarbonyle ou benzyloxycarbonyle de formule générale $COOR_2$ où $R_2$ représente un groupe alkyle en $C_1$-$C_6$ ou un groupe benzyle, soit un groupe aminocarbonyle substitué de formule générale $CONHR_3$ où $R_3$ représente un groupe alkyle en $C_1$-$C_6$ ou un groupe phényle, soit un groupe arylsulfonyle de formule générale $SO_2R_4$ où $R_4$ représente un groupe phényle,
procédé caractérisé en ce qu'on fait réagir le tétrahydro-1,2,3,4 9H-pyrido[3,4-b]indole avec un tosylate de formule générale (VIII)

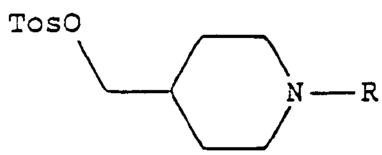

(VIII)

(dans laquelle Tos représente un groupe tosyle et R représente un groupe de formule générale $COR_1$ ou $COOR_2$) soit en l'absence soit en présence d'un solvant inerte tel que le diméthylformamide ou le xylène, à une température de 20 à 150°C, et éventuellement en présence d'une base organique, telle qu'une amine tertiaire, ou minérale telle qu'un carbonate ou hydrogénocarbonate alcalin.

**Patentansprüche**

**Patentansprüche für folgende Vertragsstaaten : AT, BE, CH, DE, FR, GB, IT, LI, LU, NL, SE**

1. Verbindungen der allgemeinen Formel (I)

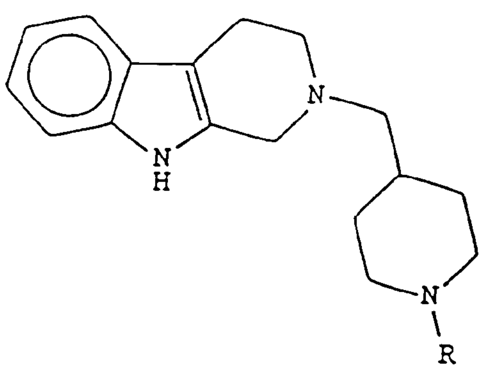

(I)

in welcher R für ein Wasserstoffatom, für eine Alkylcarbonyl-, Arylalkylcarbonyl- oder Arylcarbonylgruppe der

allgemeinen Formel $COR_1$, in welcher $R_1$ eine $C_1$-$C_6$-Alkylgruppe oder eine Benzylgruppe oder eine Phenylgruppe bedeutet, die gegebenenfalls 1 bis 3 Substituenten, ausgewählt aus Halogenatomen und den Gruppen Trifluormethyl, $C_1$-$C_3$-Alkyl und $C_1$-$C_3$-Alkoxy, trägt, für eine Alkoxycarbonyl- oder Benzyloxycarbonylgruppe der allgemeinen Formel $COOR_2$, in welcher $R_2$ eine $C_1$-$C_6$-Alkyl- oder eine Benzylgruppe darstellt, für eine substituierte Aminocarbonylgruppe der allgemeinen Formel $CONHR_3$, in welcher $R_3$ eine $C_1$-$C_6$-Alkyl- oder eine Phenylgruppe bedeutet, oder für eine Arylsulfonylgruppe der allgemeinen formel $SO_2R_4$, in welcher $R_4$ eine Phenylgruppe darstellt, steht, sowie deren Additionssalze mit pharmakologisch verwendbaren Säuren.

2. Verfahren zur Herstellung von Verbindungen nach Anspruch 1, dadurch gekennzeichnet, daß man zuerst die Piperidin-4-carbonsäure mit Benzylchloroformiat in Gegenwart einer Base in wässerigem Medium reagieren läßt, um die 1-(Benzyloxycarbonyl)-piperidin-4-carbonsäure zu erhalten, dann das 1,2,3,4-Tetrahydro-9H-pyrido[3,4-b]indol mit dem 1-(Benzyloxycarbonyl)-piperidin-4-carbonsäurechlorid (hergestellt in situ mit Hilfe von Thionylchlorid) in einem inerten Lösungsmittel in Gegenwart einer Base bei Raumtemperatur umsetzt, um den 4-[(1,2,3,4-Tetrahydro-9H-pyrido[3,4-b]indol-2-yl)-carbonyl]-piperidin-1-carbonsäurebenzylester zu erhalten, den man einer katalytischen Reduktion unterwirft, um das 2-[(Pyridin-4-yl)-carbonyl]-1,2,3,4-tetrahydro-9H-pyrido[3,4-b]indol zu gewinnen, welch letzteres anschließend einer Reduktion mit Lithiumaluminiumhydrid in einem etherischen Lösungsmittel bei einer Temperatur von 20 bis 67°C unterworfen wird, um das 2-[(Pyridin-4-yl)-methyl]-1,2,3,4-tetrahydro-9H-pyrido[3,4-b]indol zu erhalten, worauf man

— zur Herstellung einer Verbindung der allgemeinen Formel (I), in welcher R für eine Gruppe der allgemeinen Formel $COR_1$ steht, das 2-[(Pyridin-4-yl)-methyl]-1,2,3,4-tetrahydro-9H-pyrido[3,4-b]indol mit einem Säurechlorid der allgemeinen Formel $ClCOR_1$ (worin $R_1$ die in Anspruch 1 genannte Bedeutung hat) in Gegenwart einer Base in einem halogenierten Lösungsmittel bei Raumtemperatur umsetzt oder

— zur Herstellung einer Verbindung der allgemeinen Formel (I), in welcher R für eine Gruppe der allgemeinen Formel $COOR_2$ steht, das 2-[(Pyridin-4-yl)-methyl]-1,2,3,4-tetrahydro-9H-pyrido[3,4-b]indol mit einem Chloroformiat der allgemeinen Formel $ClCOOR_2$ (worin $R_2$ die in Anspruch 1 genannte Bedeutung hat) in Gegenwart einer Base in einem halogenierten Lösungsmittel bei Raumtemperatur umsetzt oder

— zur Herstellung einer Verbindung der allgemeinen Formel (I), in welcher R für eine Gruppe der allgemeinen Formel $CONHR_3$ steht, das 2-[(Pyridin-4-yl)-methyl]-1,2,3,4-tetrahydro-9H-pyrido[3,4-b]indol mit einem Isocyanat der allgemeinen Formel $R_3NCO$ (worin $R_3$ die in Anspruch 1 genannte Bedeutung hat) in einem halogenierten Lösungsmittel umsetzt oder

— zur Herstellung einer Verbindung der allgemeinen Formel (I), in welcher R für eine Gruppe der allgemeinen Formel $SO_2R_4$ steht, das 2-[(Pyridin-4-yl)-methyl]-1,2,3,4-tetrahydro-9H-pyrido[3,4-b]indol mit einem Phenylsulfonylchlorid der allgemeinen Formel $ClSO_2R_4$ (worin $R_4$ die in Anspruch 1 genannte Bedeutung hat) in Gegenwart einer Base in einem halogenierten Lösungsmittel bei Raumtemperatur umsetzt.

3. Verfahren zur Herstellung von Verbindungen nach Anspruch 1, dadurch gekennzeichnet, daß man das 1,2,3,4-Tetrahydro-9H-pyrido[3,4-b]indol mit einem Tosylat der allgemeinen Formel (VIII)

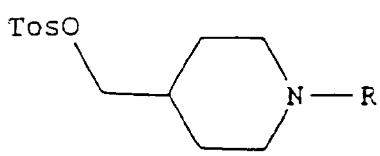

(VIII)

(in welcher Tos für ein Tosylgruppe und R für eine Gruppe der allgemeinen Formel $COR_1$ oder $COOR_2$ steht) in Ab- oder Anwesenheit eines inerten Lösungsmittels, wie Dimethylformamid oder Xylol, bei einer Temperatur von 20 bis 150°C und gegebenenfalls in Gegenwart einer organischen Base, wie einem tertiären Amin, oder einer mineralischen Base, wie einem Alkalicarbonat oder -hydrogencarbonat, umsetzt.

4. Pharmazeutische Zusammensetzung, dadurch gekennzeichnet, daß sie eine Verbindung nach Anspruch 1 in Kombination mit einem geeigneten Trägerstoff enthält.

**Patentansprüche für folgende Vertragsstaaten : ES, GR**

1. Verfahren zur Herstellung von Verbindungen der allgemeinen Formel (I)

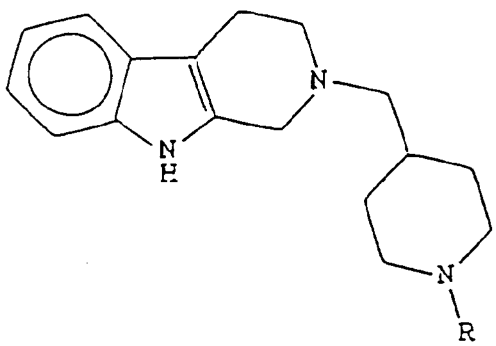

(I)

in welcher R für ein Wasserstoffatom, für eine Alkylcarbonyl-, Arylalkylcarbonyl- oder Arylcarbonylgruppe der allgemeinen Formel $COR_1$, in welcher $R_1$ eine $C_1$-$C_6$-Alkylgruppe oder eine Benzylgruppe oder eine Phenylgruppe bedeutet, die gegebenenfalls 1 bis 3 Substituenten, ausgewählt aus Halogenatomen und den Gruppen Trifluormethyl, $C_1$-$C_3$-Alkyl und $C_1$-$C_3$-Alkoxy, trägt, für eine Alkoxycarbonyl- oder Benzyloxycarbonylgruppe der allgemeinen Formel $COOR_2$, in welcher $R_2$ eine $C_1$-$C_6$-Alkyl- oder eine Benzylgruppe darstellt, für eine substituierte Amino-carbonylgruppe der allgemeinen Formel $CONHR_3$, in welcher $R_3$ eine $C_1$-$C_6$-Alkyl- oder eine Phenylgruppe bedeutet, oder für eine Arylsulfonylgruppe der allgemeinen Formel $SO_2R_4$, in welcher $R_4$ eine Phenylgruppe darstellt, steht, dadurch gekennzeichnet, daß man zuerst die Piperidin-4-carbonsäure mit Benzylchloroformiat in Gegenwart einer Base in wässerigem Medium reagieren läßt, um die 1-(Benzyloxycarbonyl)-piperidin-4-carbonsäure zu erhalten, dann das 1,2,3,4-Tetrahydro-9H-pyrido[3,4-b]indol mit dem 1-(Benzyloxycarbonyl)-piperidin-4-carbonsäurechlorid (hergestellt in situ mit Hilfe von Thionylchlorid) in einem inerten Lösungsmittel in Gegenwart einer Base bei Raumtemperatur umsetzt, um den 4-[(1,2,3,4-Tetrahydro-9H-pyrido[3,4-b]indol-2-yl)-carbonyl]-piperidin-1-carbonsäurebenzylester zu erhalten, den man einer katalytischen Reduktion unterwirft, um das 2-[(Pyridin-4-yl)-carbonyl]1,2,3,4-tetrahydro-9H-pyrido[3,4-b]indol zu gewinnen, welch letzteres anschließend einer Reduktion mit Lithiumaluminiumhydrid in einem etherischen Lösungsmittel bei einer Temperatur von 20 bis 67°C unterworfen wird, um das 2-[(Pyridin-4-yl)-methyl]-1,2,3,4-tetrahydro-9H-pyrido[3,4-b]indol zu gewinnen, worauf man

— zur Herstellung einer Verbindung der allgemeinen Formel (I), in welcher R für eine Gruppe der allgemeinen Formel $COR_1$ steht, das 2-[(Pyridin-4-yl)-methyl]-1,2,3,4-tetrahydro-9H-pyrido[3,4-b]indol mit einem Säurechlorid der allgemeinen Formel $ClCOR_1$ (worin $R_1$ die oben genannte Bedeutung hat) in Gegenwart einer Base in einem halogenierten Lösungsmittel bei Raumtemperatur umsetzt oder

— zur Herstellung einer Verbindung der allgemeinen Formel (I), in welcher R für eine Gruppe der allgemeinen Formel $COOR_2$ steht, das 2-[(Pyridin-4-yl)-methyl]-1,2,3,4-tetrahydro-9H-pyrido[3,4-b]indol mit einem Chloroformiat der allgemeinen Formel $ClCOOR_2$ (worin $R_2$ die oben genannte Bedeutung hat) in Gegenwart einer Base in einem halogenierten Lösungsmittel bei Raumtemperatur umsetzt oder

— zur Herstellung einer Verbindung der allgemeinen Formel (I), in welcher R für eine Gruppe der allgemeinen Formel $CONHR_3$ steht, das 2-[(Pyridin-4-yl)-methyl]-1,2,3,4-tetrahydro-9H-pyrido[3,4-b]indol mit einem Isocyanat der allgemeinen Formel $R_3NCO$ (worin $R_3$ die oben genannte Bedeutung hat) in einem halogenierten Lösungsmittel umsetzt oder

— zur Herstellung einer Verbindung der allgemeinen Formel (I), in welcher R für eine Gruppe der allgemeinen Formel $SO_2R_4$ steht, das 2-[(Pyridin-4-yl)-methyl]-1,2,3,4-tetrahydro-9H-pyrido[3,4-b]indol mit einem Phenylsulfonylchlorin der allgemeinen Formel $ClSO_2R_4$ (worin $R_4$ die oben genannte Bedeutung hat) in Gegenwart einer Base in einem halogenierten Lösungsmittel bei Raumtemperatur umsetzt.

2. Verfahren zur Herstellung von Verbindungen der allgemeinen Formel (I)

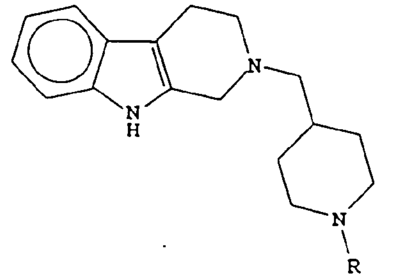

(I)

in welcher R für ein Wasserstoffatom, für eine Alkylcarbonyl-, Arylalkylcarbonyl- oder Arylcarbonylgruppe der allgemeinen Formel $COR_1$, in welcher $R_1$ eine $C_1$-$C_6$-Alkylgruppe oder eine Benzylgruppe oder eine Phenylgruppe bedeutet, die gegebenenfalls 1 bis 3 Substituenten, ausgewählt aus Halogenatomen und den Gruppen Trifluormethyl, $C_1$-$C_3$-Alkyl und $C_1$-$C_3$-Alkoxy, trägt, für eine Alkoxycarbonyl- oder Benzyloxycarbonylgruppe der allgemeinen Formel $COOR_2$, in welcher $R_2$ eine $C_1$-$C_6$-Alkyl- oder eine Benzylgruppe darstellt, für eine substituierte Amino-carbonylgruppe der allgemeinen Formel $CONHR_3$, in welcher $R_3$ eine $C_1$-$C_6$-Alkyl- oder eine Phenylgruppe bedeutet, oder für eine Arylsulfonylgruppe der allgemeinen Formel $SO_2R_4$, in welcher $R_4$ eine Phenylgruppe darstellt, steht, dadurch gekennzeichnet, daß man das 1,2,3,4-Tetrahydro-9H-pyrido[3,4-b]indol mit einem Tosylat der allgemeinen Formel (VIII)

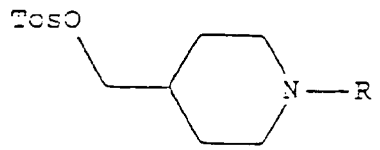

(VIII)

(in welcher Tos für ein Tosylgruppe und R für eine Gruppe der allgemeinen Formel $COR_1$ oder $COOR_2$ steht) in Ab- oder Anwesenheit eines inerten Lösungsmittels, wie Dimethylformamid oder Xylol, bei einer Temperatur von 20 bis 150°C und gegebenenfalls in Gegenwart einer organischen Base, wie einem tertiären Amin, oder einer mineralischen Base, wie einem Alkalicarbonat oder -hydrogencarbonat, umsetzt.

## Claims

## Claims for the following Contracting States : AT, BE, CH, DE, FR, GB, IT, LI, LU, NL, SE

1. Compounds of general formula (I)

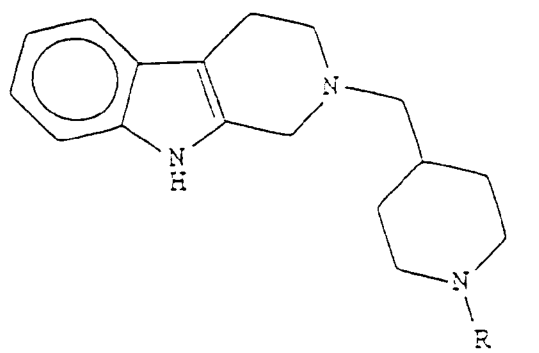

(I)

in which R denotes either a hydrogen atom or an alkylcarbonyl, arylalkylcarbonyl or arylcarbonyl group of general formula $COR_1$, where $R_1$ denotes a $C_1$-$C_6$ alkyl group or a benzyl group or a phenyl group optionally bearing from 1l to 3 substituents chosen from halogen atoms and trifluoromethyl, $C_1$-$C_3$ alkyl and $C_1$-$C_3$ alkoxy groups or an alkoxycarbonyl or benzyloxycarbonyl group of general formula $COOR_2$, where $R_2$ denotes a $C_1$-$C_6$ alkyl group or a benzyl group, or a substituted aminocarbonyl group of general formula $CONHR_3$, where $R_3$ denotes a $C_1$-$C_6$ alkyl group or a phenyl group, or an arylsulphonyl group of general formula $SO_2R_4$, where $R_4$ denotes a phenyl group, as well as their addition salts with pharmacologically acceptable acids.

2. Process for preparing the compounds according to Claim 1, characterized in that 4-piperidinecarboxylic acid is first reacted with benzylchloroformate, in the presence of a base and in an aqueous medium, to obtain 1-(benzyloxycarbonyl)-4-piperidinecarboxylic acid, 1,2,3,4-tetrahydro-9H-pyrido[3,4-b]indole is then reacted with 1-(benzyloxycarbonyl)-4-piperidinecarboxylic acid chloride (prepared in situ by means of thionyl chloride), in an inert solvent, in the presence of a base, at room temperature, to obtain benzyl 4-[(1,2,3,4-tetrahydro-9H-pyrido[3,4,-b]indol-2-yl)carbonyl]-1-piperidinecarboxylate, which is subjected to a catalytic reduction to obtain 2-[(4-piperidyl)carbonyl]-1,2,3,4-tetrahydro-9H-pyrido-[3,4-b]indole, the latter is then subjected to a reduction by means of lithium aluminium hydride in an ethereal solvent, at a temperature of 20 to 67°C, to obtain 2-[(4-piperidyl)methyl]-1,2,3,4-tetrahydro-9H-pyrido[3,4-b]-indole, and then,

— to prepare a compound of general formula (I) in which R denotes a group of general formula $COR_1$, the 2-[(4-piperidyl)methyl]-1,2,3,4-tetrahydro-9H-pyrido[3,4-b]-indole is reacted with an acid chloride of gen-

eral formula $ClCOR_1$ (where $R_1$ is as defined in Claim 1), in the presence of a base, in a halogenated solvent, at room temperature, or alternatively

— to prepare a compound of general formula (I) in which R denotes a group of general formula $COOR_2$, the 2-[(4-piperidyl)methyl]-1,2,3,4-tetrahydro-9H-pyrido[3,4,-b]-indole is reacted with a chloroformate of general formula $ClCOOR_2$ (where $R_2$ is as defined in Claim 1), in the presence of a base, in a halogenated solvent, at room temperature, or alternatively

— to prepare a compound of general formula (I) in which R denotes a group of general formula $CONHR_3$, the 2-[(4-piperidyl)methyl]-1,2,3,4-tetrahydro-9H-pyrido[3,4-b]indole is reacted with an isocyanate of general formula $R_3NCO$ (where $R_3$ is as defined in Claim 1), in a halogenated solvent, or alternatively

— to prepare a compound of general formula (I) in which R denotes a group of general formula $SO_2R_4$, the 2-[(4-piperidyl) methyl]-1,2,3,4-tetrahydro-9H-pyrido[3,4-b]indole is reacted with a phenylsulphonyl chloride of general formula $ClSO_2R_4$ (where R, is as defined in Claim 1), in the presence of a base, in a halogenated solvent, at room temperature.

3. Process for preparing the compounds according to Claim 1, characterized in that 1,2,3,4-tetrahydro-9H-pyrido[3,4-b]indole is reacted with a tosylate of general formula (VIII)

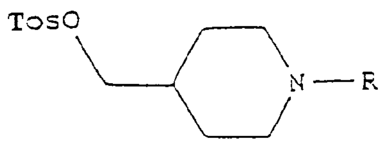

(VIII)

(in which Tos denotes a tosyl group and R denotes a group of general formula $COR_1$ or $COOR_2$), either in the absence or in the presence of an inert solvent such as direthylformamide or xylene, at a temperature of 20 to 150°C, and optionally in the presence of an organic base such as a tertiary amine or an inorganic base such as an alkali metal carbonate or hydrogen carbonate.

4. Pharmaceutical composition, characterized in that it contains a compound according to Claim 1, in combination with a suitable excipient.

**Claims for the following Contracting States : ES, GR**

1. Process for preparing compounds of general formula (I)

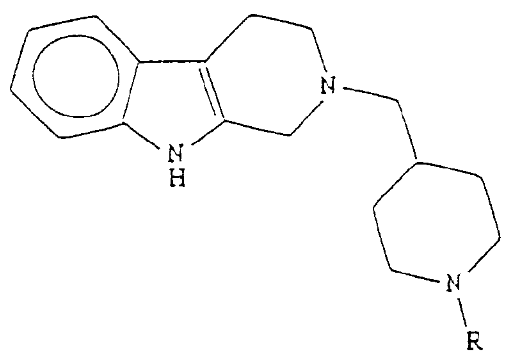

(I)

in which R denotes either a hydrogen atom or an alkylcarbonyl, arylalkylcarbonyl or arylcarbonyl group of general formula $COR_1$, where $R_1$ denotes a $C_1$-$C_6$ alkyl group or a benzyl group or a phenyl group optionally bearing from 1 to 3 substituents chosen from halogen atoms and trifluoromethyl, $C_1$-$C_3$ alkyl and $C_1$-$C_3$ alkoxy groups or an alkoxycarbonyl or benzyloxycarbonyl group of general formula $COOR_2$, where $R_2$ denotes a $C_1$-$C_6$ alkyyl group or a benzyl group, or a substituted aminocarbonyl group of general formula $CONHR_3$, where $R_3$ denotes a $C_1$-$C_6$ alkyl group or a phenyl group, or an arylsulphonyl group of general formula $SO_2R_4$, where $R_4$ denotes a phenyl group, which process is characterized in that 4-piperidinecarboxylic acid is first reacted with benzylchloroformate, in the presence of a base and in an aqueous medium, to obtain 1-(benzyloxycarbonyl)-4-piperidinecarboxylic acid, 1,2,3,4-tetrahydro-9H-pyrido[3,4-b]-indole is then reacted with 1-(benzyloxycarbonyl)-4-piperidinecarboxylic acid chloride (prepared in situ by means of thionyl chloride), in an inert solvent, in the presence of a base, at room temperature, to obtain benzyl 4-[(1,2,3,4-tetrahydro-9H-pyrido[3,4-b]indol-2-yl)carbonyl]-1-piperidinecarboxylate, which is subjected to a catalytic reduction to obtain 2-[(4-piperidyl)carbonyl]-1,2,3,4-tetrahydro-9H-pyrido-[3,4-b]indole, the latter is then subjected to a reduction

by means of lithium aluminium hydride in an ethereal solvent, at a temperature of 20 to 67°C, to obtain 2-[(4-piperidyl)methyl]-1,2,3,4-tetrahydro-9H-pyrido[3,4-b]indole, and then,

— to prepare a compound of general formula (I) in which R denotes a group of general formula $COR_1$, the 2-[(4-piperidyl)methyl]-1,2,3,4-tetrahydro-9H-pyrido[3,4-b]indole is reacted with an acid chloride of general formula $ClCOR_1$ (where $R_1$ is as defined above), in the presence of a base, in a halogenated solvent, at room temperature, or alternatively

— to prepare a compound of general formula (T) in which R denotes a group of general formula $COOR_2$, the 2-[(4-piperidyl)methyl]-1,2,3,4-tetrahydro-9H-pyrido[3,4,-b]indole is reacted with a chloroformate of general formula $ClCOOR_2$ (where $R_2$ is as defined above), in the presence of a base, in a halogenated solvent, at room temperature, or alternatively

— to prepare a compound of general formula (I) in which R denotes a group of general formula $CONHR_3$, the 2-[(4-piperidyl)methyl]-1,2,3,4-tetrahydro-9H-pyrido[3,4-b]indole is reacted with an isocyanate of general formula $R_3NCO$ (where $R_3$ is as defined above), in a halogenated solvent, or alternatively

— to prepare a compound of general formula (I) in which R denotes a group of general formula $SO_2R_4$, the 2-[(4-piperidyl)methyl]-1,2,3,4-tetrahydro-9H-pyrido[3,4-b]indole is reacted with a phenylsulphonyl chloride of general formula $ClSO_2R_4$ (where $R_4$ is as defined above), in the presence of a base, in a halogenated solvent, at room temperature.

2. Process for preparing compounds of general formula (I)

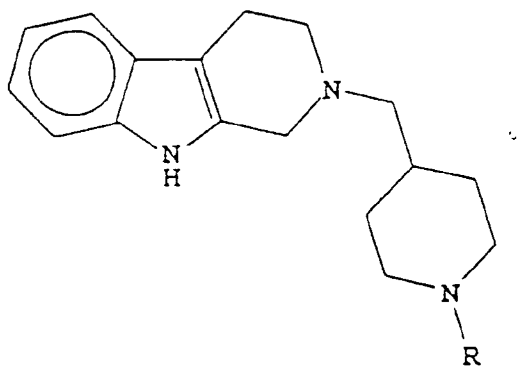

(I)

in which R denotes in which R denotes either a hydrogen atom or an alkylcarbonyl, arylalkylcarbonyl or arylcarbonyl group of general formula $COR_1$, where $R_1$ denotes a $C_1$-$C_6$ alkyl group or a benzyl group or a phenyl group optionally bearing from 1 to 3 substituents chosen from halogen atoms and trifluoromethyl, $C_1$-$C_3$ alkyl and $C_1$-$C_3$ alkoxy groups or an alkoxycarbonyl or benzyloxycarbonyl group of general formula $COOR_2$, where $R_2$ denotes a $C_1$-$C_6$ alkyl group or a benzyl group, or a substituted amino-carbonyl group of general formula $CONHR_3$, where $R_3$ denotes a $C_1$-$C_6$ alkyl group or a phenyl group, or an arylsulphonyl group of general formula $SO_2R_4$, where $R_4$ denotes a phenyl group, which process is characterized in that 1,2,3,4-tetrahydro-9H-pyrido[3,4-b]indole is reacted with a tosylate of general formula (VIII)

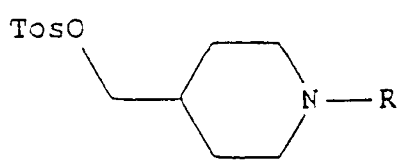

(VIII)

(in which Tos denotes a tosyl group and R denotes a group of general formula $COR_1$ or $COOR_2$), either in the absence or in the presence of an inert solvent such as dimethylformamide or xylene, at a temperature of 20 to 150°C, and optionally in the presence of an organic base such as a tertiary amine or an inorganic base such as an alkali metal carbonate or hydrogen carbonate.